(19) Europäisches Patentamt
European Patent Office
Office européen des brevets

(11) Publication number: 0 067 471 B1

# (12) EUROPEAN PATENT SPECIFICATION

(45) Date of publication of patent specification: 06.11.85

(51) Int. Cl.$^4$: C 07 D 307/79, C 07 D 413/04, A 01 N 43/82, A 01 N 47/24

(21) Application number: 82200659.9

(22) Date of filing: 27.05.82

## (54) 7-Substituted 2,3-dihydrobenzofurans, their preparation and their use as pesticides or as chemical intermediates.

(30) Priority: 15.06.81 US 273833
22.06.81 US 276303

(43) Date of publication of application:
22.12.82 Bulletin 82/51

(45) Publication of the grant of the patent:
06.11.85 Bulletin 85/45

(84) Designated Contracting States:
BE CH DE FR GB IT LI NL

(56) References cited:
EP-A-0 048 040
US-A-3 547 955
US-A-4 150 142

(73) Proprietor: SHELL INTERNATIONALE RESEARCH MAATSCHAPPIJ B.V.
Carel van Bylandtlaan 30
NL-2596 HR Den Haag (NL)

(72) Inventor: Pilgram, Kurt Hans
2607 Warwick Lane
Modesto California 95350 (US)
Inventor: Skiles, Richard Daniel
2912 Debbie Lane
Modesto California 95350 (US)

(74) Representative: Hunter, Keith Roger Ian et al
4 York Road
London SE1 7NA (GB)

EP 0 067 471 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to 7-substituted 2,3-dihydrobenzofurans, their preparation, and their use as pesticides or as intermediates in the preparation of pesticidally active compounds.

According to the invention there are provided 7-substituted 2,3-dihydrobenzofurans of general formula

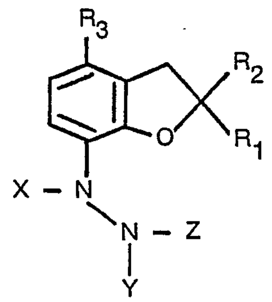

(A)

wherein X, Y and Z together represent a bridging group —CO—O—C(OR)=, or X is a chlorocarbonyl group, Y is hydrogen and Z is a carboxylate ester group —COOR; R represents a $C_{(1-2)}$ alkyl group; $R_1$ represents a $C_{(1-3)}$ alkyl group; $R_2$ represents a $C_{(1-3)}$ alkyl group or a hydrogen atom; and $R_3$ represents a $C_{(1-3)}$ alkyl group or a chlorine or bromine atom.

Those compounds wherein each of R, $R_1$, $R_2$ and $R_3$ represents a methyl group are particularly preferred because of their pesticidal activity.

In accordance with a further aspect of the invention, compounds of formula A may be prepared by a process which comprises reacting an ester of formula

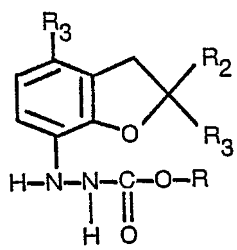

(B)

wherein R, $R_1$, $R_2$ and $R_3$ are as defined above, with phosgene in an inert solvent to yield a compound of formula A wherein X is a chlorocarbonyl group, Y is hydrogen and Z is a carboxylate ester group—COOR, followed, when the desired product is a compound of formula A wherein X, Y and Z together represent a bridging group —CO—O—C(OR)=, by treating said chlorocarbonyl compound with a base.

Reaction of the ester of formula B with phosgene is effected in an inert solvent, such as benzene, conveniently at ambient temperature. Treatment of the resulting compound of formula A wherein X is a chlorocarbonyl group, Y is hydrogen and Z is a carboxylate ester group with a base may conveniently be effected in the presence of a liquid alkanol, such as methanol, as solvent and at a temperature ranging from ambient temperature to the reflux temperature of the reaction mixture. Suitable bases include tertiary amine bases. Ethyldiisopropylamine and triethylamine are examples of preferred bases.

The esters of formula B may be prepared by reacting 2,3-dihydro-2,2,4-trialkyl-7-hydrazinobenzofuran or an acid-addition salt thereof, with a chloroformate ester of formula:

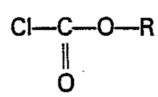

(C)

where R is as defined above in an inert solvent, such as tetrahydrofuran, in the presence of a base, for example a tertiary amine such as triethylamine or ethyldiisopropylamine.

The invention also comprehends a pesticidal composition comprising a 7-substituted 2,3-dihydrobenzofuran of formula A in association with an inert carrier therefor. Such a composition is prepared by bringing the compound of formula A into association with the inert carrier. The invention also provides a method of combating pests at a locus which comprises applying to the locus such a compound of formula A or a pesticidal composition as defined above.

The term "carrier" as used herein means a solid or fluid material, which may be inorganic or organic and of synthetic or natural origin, with which the active compound is mixed or formulated to facilitate its application to the locus to be treated, or its storage, transport or handling.

Suitable solid carriers are natural and synthetic clays and silicates, for example, natural silicas such as diatomaceous earths; magnesium silicates, for example talcs; magnesium aluminium silicates, for example, attapulgites and vermiculites; aluminium silicates, for example, kaolinites, montmorillonites and

micas; calcium carbonates; calcium sulphate; synthetic hydrated silicon oxides and synthetic calcium or aluminium silicates; elements such as, for example, carbon and sulphur; natural and synthetic resins such as, for example, coumarone resins, polyvinyl chloride and styrene polymers and copolymers; solid polychlorophenols; bitumen; waxes such as, for example, beeswax, paraffin wax, and chlorinated mineral waxes; and solid fertilizers, for example, superphosphates.

Examples of suitable fluid carriers are water, alcohols, such as for example, isopropanol; glycols; ketones such as for example, acetone, methyl ethyl ketone, methyl isobutyl ketone and cyclohexanone; ethers; aromatic hydrocarbons such as for example, benzene, toluene and xylene; petroleum fractions such as for example, kerosene, light mineral oils; chlorinated hydrocarbons, such as for example, carbon tetrachloride, perchloroethylene, trichloroethane, including liquified normally vaporous gaseous compounds. Mixtures of different liquids are often suitable. The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium or calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, or sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions may be formulated as wettable powders, dusts, granules, solutions, emulsifiable concentrates, emulsions, suspension concentrates and aerosols. Wettable powders are usually compounded to contain 25, 50 or 75% by weight and usually contain in addition to solid carrier, 3—10% by weight of a dispersing agent, 15% of a surface-active agent and where necessary, 0—10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 1/2—10% by weight of toxicant. Granules are usually prepared to have a size between 10 and 100 BS mesh (1.676—0.152 mm), and may be manufactured by agglomeration or impregnation techniques. Generally, granules will contain 1/2—25% by weight toxicant and 0—1% by weight of additives such as stabilizers, slow release modifiers and binding agents. Emulsifiable concentrates usually contain, in addition to the solvent and, when necessary, cosolvent, 10—50% weight per volume toxicant, 2—20% weight per volume emulsifers and 0—20% weight per volume of appropriate additives such as stabilizers, penetrants and corrosion inhibitors. Suspension concentrates are compounded so as to obtain a stable, non-sedimenting, flowable product and usually contain 10—75% weight toxicant, 0.5—5% weight of dispersing agents, 1—5% of surface-active agent, 0.1—10% weight of suspending agents, such as defoamers, corrosion inhibitors, stabilizers, penetrants and stickers, and as carrier, water or an organic liquid in which the toxicant is substantially insoluble; certain organic solids or inorganic salts may be dissolved in the carrier to assist in preventing sedimentation or as antifreeze agents for water.

Aqueous dispersions and emulsions, for example, compositions obtained by diluting a wettable powder or a concentrate, also are contemplated. The said emulsions may be of the water-in-oil or the oil-in-water type, and may have a thick mayonnaise-like consistency.

The compositions may also contain other ingredients, for example, other compounds possessing pesticidal, especially insecticidal, acaricidal, herbicidal or fungicidal properties.

The method of applying the pesticidal compound to control pests comprises applying it, ordinarily in a composition of one of the aforementioned types, to a locus to be protected from the pests, such as the foliage and/or the fruit of plants. It is of course applied in an amount sufficient to exert the desired action. This dosage is dependent upon many factors, including the carrier employed, the method and conditions of the application, whether the formulation is present at a locus in the form of an aerosol, or as a film, or as discrete particles, or the thickness of film or size of particles. In general, however, the effective dosage of the pesticidal compound at the locus to be protected — i.e., the dosage which the pests contact — is of the order of 0.001 to 0.05% based on the total weight of the formulation, though under some circumstances the effective concentrations will be as little as 0.0001% or as much as 2%, on the same basis.

The invention will be further understood from the following Examples, in which the identity of each precursor, and of each product, was confirmed by appropriate chemical and spectral analyses.

## Example 1

2-(2-chlorocarbonyl)-2-(2,3-dihydro-2,2,4-trimethylbenzofuran-7-yl)-hydrazinecarboxylic acid methyl ester (*1*)

3 g of a commercial 10% palladium-on-charcoal catalyst was added to a solution of 82.2 g of 2,3-dihydro-2,2,4-trimethyl-7-nitrobenzofuran (prepared by the method described in US—A—3,412,110) in 500 ml of tetrahydrofuran. The resulting mixture was treated with hydrogen (50 p.s.i.g.) in a Parr shaker, then

filtered. The solvent was evaporated from the filtrate. The residue was dissolved in one liter of anhydrous ether cooled to 0°C. The solution was treated with a slight excess of gaseous anhydrous hydrogen chloride.

The resulting mixture was filtered and the solid was dried to give 7-amino-2,3-dihydro-2,2,4-trimethylbenzofuran hydrochloride (*1A*), as a white solid.

A chilled (5°C) solution of 82.7 g of *1A*, 146 ml of concentrated hydrochloric acid and 450 ml of water was diazotized by the drop-by-drop addition over a 30-minute period of a solution of 31.5 g sodium nitrite in 45 ml of water. After filtration, the resulting solution was stirred at 5°C for 45 minutes and then added drop-by-drop to a stirred solution of 367 g of sodium sulfite in 705 ml of water, at 5°C. The resulting mixture was stirred for 2 hours at room temperature. Then a slurry of 72.2 g of sodium dithionite in 100 ml of water was added in portions. The resulting mixture was stirred for 2 hours at room temperature, then heated at 70°C for 10 minutes, and 500 g of potassium chloride was added. After 18 hours, the resulting mixture was cooled at 5°C and filtered. The solid was dried to give the potassium salt of (2,3-dihydro-2,2,4-trimethylbenzofuran-7-yl)hydrazinesulfonic acid (*1B*), as an off-white solid.

18 g of hydrogen chloride was introduced into a stirred slurry of 56 g of *1B* in 250 ml of ethanol. The resulting mixture was stirred for 3 hours, and the solvent was evaporated. The residue was dissolved in 50 ml of water, the solution was made basic with aqueous sodium hydroxide, and extracted with ether. The extract was washed with cold water, dried ($MgSO_4$), and filtered, and the solvent was evaporated to give 2,3-dihydro-2,2,4-trimethyl-7-hydrazinobenzofuran (*1C*) as a dark oil.

17.8 g of *1C* thus prepared and 12.9 g of ethyldiisopropylamine were dissolved in 200 ml of tetrahydrofuran. 9.45 g of methyl chloroformate was added drop-by-drop to the stirred solution, and the resulting mixture was stirred for one hour. The solvent then was evaporated, the residue was washed with water, extracted with ether, and the extract was dried. Part of the ether was evaporated from the extract and the remaining solution was cooled. The resulting solid was collected to give the methyl ester of 2-(2,3-dihydro-2,2,4-trimethylbenzofuran-7-yl)hydrazinecarboxylic acid (*1D*), as a solid, m.p.; 125—128°C.

A solution of 6.3 g of phosgene in 60 ml of benzene was added to a solution of 13.5 g of *1D* in 100 ml benzene at room temperature. The resulting mixture was held for 24 hours at room temperature, and the solvent was evaporated to give *1*, as an oil.

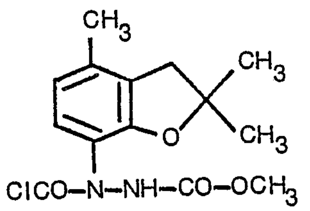

(1)

Following similar procedures to those detailed above, similar chlorocarbonyl compounds can be obtained in which R, $R_1$ and $R_2$ are all methyl and $R_3$ is isopropyl, and in which R is methyl and $R_1$, $R_2$ and $R_3$ are all ethyl. Similar compounds wherein $R_3$ is halogen may be prepared in the same manner from the appropriate hydrazinecarboxylic acid ester substituted at the 4-position of the benzofuranyl moiety by the halogen.

Example 2

3-(2,3-dihydro-2,2,4-trimethylbenzofuran-7-yl)-5-methoxy-1,3,4-oxadiazol-2(3H)-one (*2*)

A solution of 13 g of *1* and 15 g of ethyldiisopropylamine in 200 ml of methanol was refluxed for 15 minutes. After cooling, the solvent was evaporated. The residue was triturated with water, acidified with hydrochloric acid and extracted with ether. The extract was dried ($MgSO_4$) and the solvent was evaporated. The residue was crystallized from ether-hexane to give *2*, as a solid, m.p.: 60—62°C.

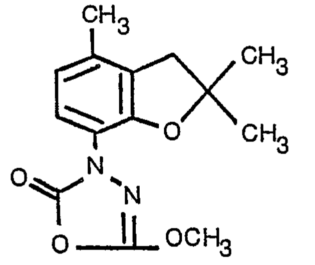

(2)

Following similar procedures to those detailed above, similar oxadiazolone compounds can be obtained in which R, $R_1$ and $R_2$ are all methyl and $R_3$ is isopropyl, and in which R is methyl and $R_1$, $R_2$ and $R_3$ are all ethyl. Similar compounds wherein $R_3$ is halogen may be prepared in the same manner from the appropriate chlorocarbonyl hydrazinecarboxylic acid ester substituted at the 4-position of the benzofuranyl moiety by the halogen.

Activity of compounds of this invention with respect to insect and acarine pests was determined by using standardized test methods to measure the toxicity of the compounds as follows:

1. Houseflies (*Musca domestica* (Linne)) were tested by placing 50 4- to 5-day old houseflies into a spray cage and spraying with 0.6 ml of a solution of test compound. After spraying, the flies were anesthetized with $CO_2$ and transferred to a recovery cage containing a milk pad for food.

The cages were held for 18—20 hours after which mortality counts were made. Both dead and moribund flies were counted. The tests were conducted employing several dosage rates for each test compound.

2. Pea aphids (*Acyrthosiphon pisum* (Harris)) were tested by placing about 100 aphids on broad bean plants. The plants were sprayed with dilutions of an acetone solution of the test compound in water containing an emulsifier and held in containers under laboratory conditions for 18 to 20 hours, at which time the living aphids in the containers were counted. The tests were conducted employing several different dosage rates for each test compound.

3, Adult female two-spotted spider mites (*Tetranychus urticae* (Koch)) were tested by placing 50—75 mites on the bottom side of leaves of pinto bean plants. The leaves were sprayed with dilutions of an acetone solution of the test compound in water containing an emulsifier and kept under laboratory conditions for about 20 hours, at which time mortality counts were made. The tests were conducted employing several different dosage rates for each test compound.

4. Mosquito larvae (*Anophelus albimanus* (Weide)) were tested by placing 10 living and active mosquito larvae in a jar containing a 0.1 ml aliquot of 1% acetone solution of the test compound thoroughly mixed with 100 ml of distilled water. After 18—22 hours, mortality counts were taken. Both dead and moribund larvae were counted as dead. Larvae which did not swim after being prodded with a needle were considered moribund. The tests were conducted employing several different dosage rates for each compound.

5. Corn earworm larvae (*Heliothis zea* (Boddie)) were tested by spraying a broad bean plant with dilutions of an acetone solution of the test compound in water containing an emulsifier. Immediately after spraying, 5 larvae were transferred to the plant and held for 44—46 hours, at which time the dead and moribund larvae were counted. The tests were conducted employing several different dosage rates for each test compound.

In the case of each species of insect, the concentration of the test compound in the formulation required to kill fifty percent of the test insects. — i.e., the $LC_{50}$ dosage — was determined. The results are set out in Table I, $LC_{50}$ figures being expressed in parts per million.

TABLE 1

| Compound | $LC_{50}$ (ppm) | | | | |
|---|---|---|---|---|---|
| | Housefly | Pea Aphid | Corn Earworm | Spidermite | Mosquito larvae |
| 1 | >0.5 | 0.0002 | 0.033 | >0.2 | 0.02 |
| 2 | 0.02 | 0.00003 | 0.024 | 0.035 | 0.01 |

**Claims**

1. A 7-substituted 2,3-dihydrobenzofuran of general formula

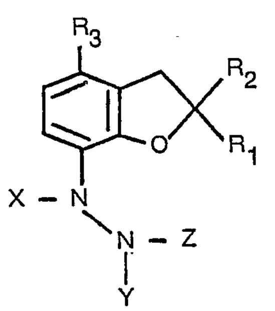

(A)

wherein X, Y and Z together represent a bridging group —CO—O—C(OR)=, or X is a chlorocarbonyl group, Y is hydrogen and Z is a carboxylate ester group —COOR; R represents a $C_{(1-2)}$ alkyl group; $R_1$ represents a

$C_{(1-3)}$ alkyl group; $R_2$ represents a $C_{(1-3)}$ alkyl group or a hydrogen atom; and $R_3$ represents a $C_{(1-3)}$ alkyl group or a chlorine or bromine atom.

2. A 7-substituted 2,3-dihydrobenzofuran as claimed in claim 1, wherein each of R, $R_1$, $R_2$ and $R_3$ represents a methyl group.

3. 3-(2,3-dihydro-2,2,4-trimethylbenzofuran-7-yl)-5-methoxy-1,3,4-oxadiazol-2(3H)-one, having the formula (2):—

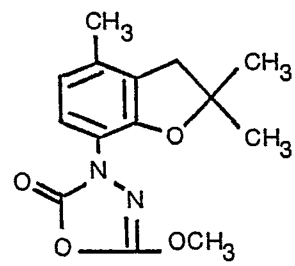

(2)

4. A process for preparing a 7-substituted 2,3-dihydrobenzofuran of formula A as defined in any of claims 1 to 3 which comprises reacting an ester of formula:

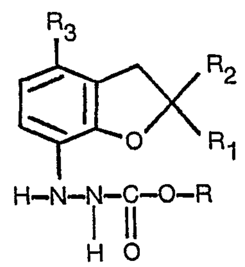

(B)

wherein R, $R_1$, $R_2$ and $R_3$ are as defined in claim 1, with phosgene in an inert solvent to yield a compound of formula A wherein X is a chlorocarbonyl group, Y is hydrogen and Z is a carboxylate ester group—COOR, followed, when the desired product is a compound of formula A wherein X, Y and Z together represent a bridging group —CO—O—C(OR)=, by treating the said chlorocarbonyl compound with a base.

5. A pesticidal composition comprising a 7-substituted 2,3-dihydrobenzofuran of formula A as defined in claims 1, 2 or 3 in association with an inert carrier therefor.

6. A method of combating pests at a locus which comprises applying to the locus a 7-substituted 2,3-dihydrobenzofuran of formula A as defined in claims 1, 2 or 3 or a composition according to claim 5.

**Patentansprüche**

1. Ein in der 7-Stellung substituiertes 2,3-Dihydrobenzofuran der allgemeinen Formel

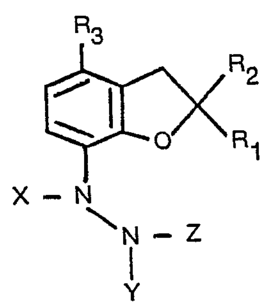

(A)

in welcher X, Y und Z zusammen eine Brückengruppe —CO—O—C(OR)= bilden, oder X eine Chlorcarbonylgruppe ist, Y Wasserstoff und Z eine Carbonsäureestergruppe —COOR bedeutet; R eine $C_{(1-2)}$-Alkylgruppe darstellt; $R_1$ eine $C_{(1-3)}$-Alkylgruppe bedeutet; $R_2$ eine $C_{(1-3)}$-Alkylgruppe oder ein Wasserstoffatom ist; und $R_3$ eine $C_{(1-3)}$-Alkylgruppe oder ein Chlor- oder Bromatom darstellt.

2. Ein in der 7-Stellung substituiertes 2,3-Dihydrobenzofuran wie in Anspruch 1 beansprucht, wobei jede der Gruppen R, $R_1$, $R_2$ und $R_3$ eine Methylgruppe darstellt.

3. 3-(2,3-Dihydro-2,2,4-trimethylbenzofuran-7-yl)-5-methoxy-1,3,4-oxadiazol-2(3H)-on der Formel (2):

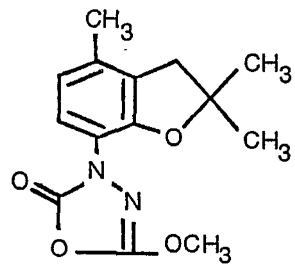

(2)

4. Ein Verfahren zur Herstellung eines in der 7-Stellung substituierten 2,3-Dihydrobenzofurans der Formel A wie in einem der Ansprüche 1 bis 3 definiert, welches Verfahren die Umsetzung eines Esters der Formel

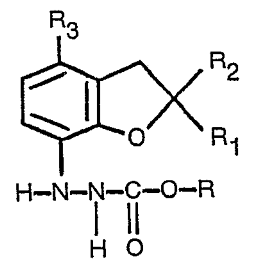

(B)

in welcher R, $R_1$, $R_2$ und $R_3$ wie in Anspruch 1 definiert sind, mit Phosgen in einem inerten Lösungsmittel zwecks Erhalt einer Verbindung der Formel A, in welcher X eine Chlorcarbonylgruppe, Y Wasserstoff und Z eine Carbonsäureestergruppe —COOR bedeutet, und anschließend, wenn das gewünschte Produkt eine Verbindung der Formel A ist, in welcher X, Y und Z zusammen eine Brückengruppe —CO—O—C(OR)= bilden, das Behandeln dieser Chlorcarbonylverbindung mit einer Base, umfaßt.

5. Eine Pestizid-Zusammensetzung umfassend ein in der 7-Stellung substituiertes 2,3-Dihydrobenzofuran der Formel A wie in Anspruch 1, 2 oder 3 definiert in Verbindung mit einem inerten Träger dafür.

6. Ein Verfahren zur lokalen Schädlingsbekämpfung, welches das Aufbringen eines in der 7-Stellung substituierten 2,3-Dihydrobenzofurans der Formel A wie in Anspruch 1, 2 oder 3 beansprucht oder einer Zusammensetzung nach Anspruch 5 auf die betreffende Stelle, umfaßt.

**Revendications**

1. Un 2,3-dihydrobenzofuranne substitué en position 7 de formule générale

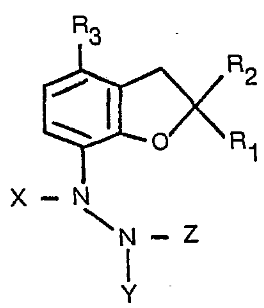

(A)

où X, Y et Z représentent ensemble un groupe formant pont —CO—O—C(OR)=, ou X est un groupe chlorocarbonyle, Y est de l'hydrogène et Z est un groupe ester carboxylate —COOR; R représente un groupe alcoyle en $C_{(1-2)}$; $R_1$ représente un groupe alcoyle en $C_{(1-3)}$; $R_2$ représente un groupe alcoyle en $C_{(1-3)}$ ou un atome d'hydrogène; et $R_3$ représente un groupe alcoyle en $C_{(1-3)}$ ou un atome de chlore ou de brome.

2. Un 2,3-dihydrobenzofuranne substitué en position 7 selon la revendication 1, où R, $R_1$, $R_2$ et $R_3$ representent chacun un groupe méthyle.

3. La 3-(2,3-dihydro-2,2,4-triméthylbenzofuran-7-yl)-5-méthoxy-1,3,4-oxadiazol-2(3H)-one, ayant la formule (2):

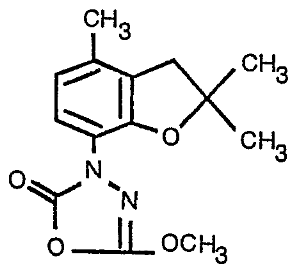

(2)

4. Un procédé pour la préparation d'un 2,3-dihydrobenzofuranne substitué en position 7 de formule A tel que défini dans l'une quelconque des revendications 1 à 3, qui comprend la réaction d'un ester de formule:

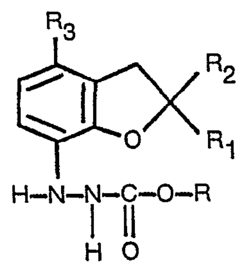

(B)

où R, $R_1$, $R_2$ et $R_3$ sont tels que définis dans la revendication 1, avec du phosgène dans un solvant inerte pour donner un composé de formule A où X est un groupe chlorocarbonyle, Y est de l'hydrogène et Z est un groupe ester carboxylate, —COOR, suivie, quand le produit désiré est un composé de formule A où X, Y et Z représentent ensemble un groupe formant pont —CO—O—C(OR)=, du traitement du composé chlorocarbonylé par une base.

5. Une composition pesticide comprenant un 2,3-dihydrobenzofuranne substitué en position 7 de formule A tel que défini dans les revendications 1, 2 ou 3 en association avec un véhicule inerte approprié.

6. Un procédé de lutte contre des organismes nuisibles en un lieu, qui comprend l'application au lieu concerné d'un 2,3-dihydrobenzofuranne substitué en position 7 de formule A tel que défini dans les revendications 1, 2 ou 3 ou d'une composition selon la revendication 5.